# EUROPEAN PATENT APPLICATION

(11) **EP 2 893 868 A1**
(43) Date of publication of application: **15.07.2015**
(21) Application number: 14180497.1
(22) Date of filing: 11.08.2014
(51) Int. Cl.: A61B 5/00, A61B 8/00, A61B 8/08

(54) **Photoacoustic apparatus and method of operating same**

(30) Priority: 14.01.2014 KR 20140004688
(71) Applicant: Samsung Medison Co., Ltd., Gangwon-do 250-870 (KR); Samsung Electronics Co., Ltd, Gyeonggi-do 443-742 (KR)
(72) Inventor: Oh, Jung-taek, Seoul (KR); Jung, Jong kyu, Gangwon-do (KR); Kim, Jung-ho, Gangwon-do (KR); Koh, Dal-kwon, Gangwon-do (KR)
(74) Representative: Schmid, Wolfgang

(57) **Abstract**

A photoacoustic (PA) apparatus and a method of operating the same are provided. The method includes: irradiating a laser beam onto a region of interest (ROI) which includes a flow and receiving a first PA signal corresponding to the irradiated laser beam; generating a first PA image on the basis of the first PA signal; irradiating a laser beam onto the ROI where the flow is restricted and receiving a second PA signal corresponding to the irradiated laser beam; generating a second PA image on the basis of the second PA signal; generating a difference image between the first PA image and the second PA image; and displaying the difference image.

## Description

### RELATED APPLICATIONS

This application claims the benefit of Korean Patent Application No. 10-2014-0004688, filed on January 14, 2014, in the Korean Intellectual Property Office, the disclosure of which is incorporated herein in its entirety by reference.

### BACKGROUND

### 1. Field

One or more embodiments of the present invention relate to a photoacoustic (PA) apparatus and a method of operating the same, and more particularly, to a PA apparatus capable of acquiring a PA image from which an artifact has been removed and a method of operating the same.

### 2. Description of the Related Art

A PA apparatus may acquire an image of the inside of an object by irradiating a laser beam onto the object and receiving a PA signal generated by a target inside the object which absorbs the laser light.

The existing ultrasound diagnosis apparatus may image a biological structure, showing, for example, a position, a shape, and the like, and biomechanical properties of a target inside an object by irradiating an ultrasound signal generated by a transducer of a probe onto the object and receiving information on an echo signal reflected from the target.

Meanwhile, with respect to a PA image, a chemical component difference and optical characteristics of a target to be measured may be determined.

### SUMMARY

One or more embodiments of the present invention include a photoacoustic (PA) apparatus for acquiring a high quality PA image by removing an artifact therefrom and a method of operating the same.

Additional aspects will be set forth in part in the description which follows and, in part, will be apparent from the description, or may be learned by practice of the presented embodiments.

According to one or more embodiments of the present invention, a method of operating a photoacoustic (PA) apparatus includes: irradiating a laser beam onto a region of interest (ROI) which includes a flow and receiving a first PA signal corresponding to the irradiated laser beam; generating a first PA image on the basis of the first PA signal; irradiating a laser beam onto the ROI where the flow is restricted and receiving a second PA signal corresponding to the irradiated laser beam; generating a second PA image on the basis of the second PA signal; generating a difference image between the first PA image and the second PA image; and displaying the difference image.

Magnitudes of the first PA signal and the second PA signal may be proportional to an amount of the flow.

The first PA signal may include a signal corresponding to an artifact and a signal corresponding to the flow.

The second PA signal may include a signal corresponding to an artifact.

The second PA image may be an artifact image.

The difference image may be an image from which the artifact image has been removed.

The signal corresponding to the flow, which is included in the first PA signal, may be greater than the signal corresponding to an artifact, which is included in the second PA signal.

The method may further include: transmitting an ultrasound signal to the ROI and receiving an echo signal reflected from the ROI; and generating an ultrasound image on the basis of the echo signal.

The displaying of the difference image may include overlapping and displaying the difference image and the ultrasound image.

According to one or more embodiments of the present invention, a photoacoustic (PA) apparatus includes: a probe for irradiating a laser beam onto a region of interest (ROI) which includes a flow; a signal reception unit for receiving a first PA signal corresponding to the laser beam irradiated onto the ROI which includes the flow and receiving a second PA signal corresponding to the laser beam irradiated onto the ROI where the flow is restricted; an image generation unit for generating a first PA image on the basis of the first PA signal, generating a second PA image on the basis of the second PA signal, and generating a difference image between the first PA image and the second PA image; and a display unit for displaying the difference image.

The probe may transmit an ultrasound signal to the ROI, the signal reception unit may receive an echo signal reflected from the ROI, and the PA apparatus may further include an ultrasound image generation unit for generating an ultrasound image on the basis of the echo signal.

The display unit may display the ultrasound image.

The display unit may overlap and display the difference image and the ultrasound image.

### BRIEF DESCRIPTION OF THE DRAWINGS

These and/or other aspects will become apparent and more readily appreciated from the following description of the embodiments, taken in conjunction with the accompanying drawings in which:
FIG. 1 illustrates a photoacoustic (PA) image including an artifact;
FIG. 2 is a block diagram of a PA apparatus according to an embodiment of the present invention;
FIG. 3 is a block diagram of a PA apparatus according to another embodiment of the present invention;
FIG. 4 is a flowchart of a method of operating a PA apparatus, according to an embodiment of the present invention;
FIG. 5 illustrates PA signals with respect to time, which correspond to a sentinel lymph node (SLN) and an artifact;
FIGS. 6A to 6C illustrate a first PA image, a second PA image, and a difference image, respectively, according to an embodiment of the present invention; and
FIGS. 7 to 9 illustrate a PA image displayed on a display unit, according to an embodiment of the present invention.

### DETAILED DESCRIPTION

Although general terms as currently widely used are selected as much as possible as the terms used in the present invention while taking functions in the present invention into account, they may vary according to an intention of one of ordinary skill in the art, judicial precedents, or the appearance of new technology. In addition, in specific cases, terms intentionally selected by the applicant may be used, and in this case, the meaning of the terms will be disclosed in a corresponding description of the invention. Accordingly, the terms used in the present invention should be defined not by simple names of the terms but by the meaning of the terms and the contents over the present invention.

In the specification, when a certain part "includes" a certain component, this indicates that the part may further include another component instead of excluding another component unless there is different disclosure. In addition, the term, such as "...unit" or "module," disclosed in the specification indicates a unit for processing at least one function or operation, and this may be implemented by hardware, software, or a combination thereof.

In the specification, "image" indicates an image of an object, which is acquired by a photoacoustic (PA) apparatus. In addition, the object may include a human being, a creature, or a portion of the human being or the creature. For example, the object may include an organ, such as a liver, a heart, a womb, a brain, a breast, or an abdomen, or a blood vessel. In addition, the object may include a phantom, and the phantom may indicate matter having a volume that is approximate to a density and an effective atomic number of an organism.

In addition, the image may include an ultrasound image and a PA image. The ultrasound image may be an image acquired by transmitting ultrasound waves to an object and receiving an echo signal reflected from the object. The PA image may be an image acquired by irradiating light (e.g., a laser beam) onto an object and receiving a PA signal from the object.

The ultrasound image may be variously implemented. For example, the ultrasound image may be at least one selected from among the group consisting of an amplitude (A) mode image, a brightness (B) mode image, a color (C) mode image, and a Doppler (D) mode image.

According to an embodiment of the present invention, the image may be a two-dimensional (2D) image or a 3D image.

In the specification, "user" may indicate a medical expert, e.g., a medical practitioner, a nurse, a clinical pathologist, a medical image expert, or the like, or may indicate a technician for repairing medical devices but is not limited thereto.

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects of the present description. As used herein, the term "and/or" includes any and all combinations of one or more of the associated listed items. Expressions such as "at least one of," when preceding a list of elements, modify the entire list of elements and do not modify the individual elements of the list.

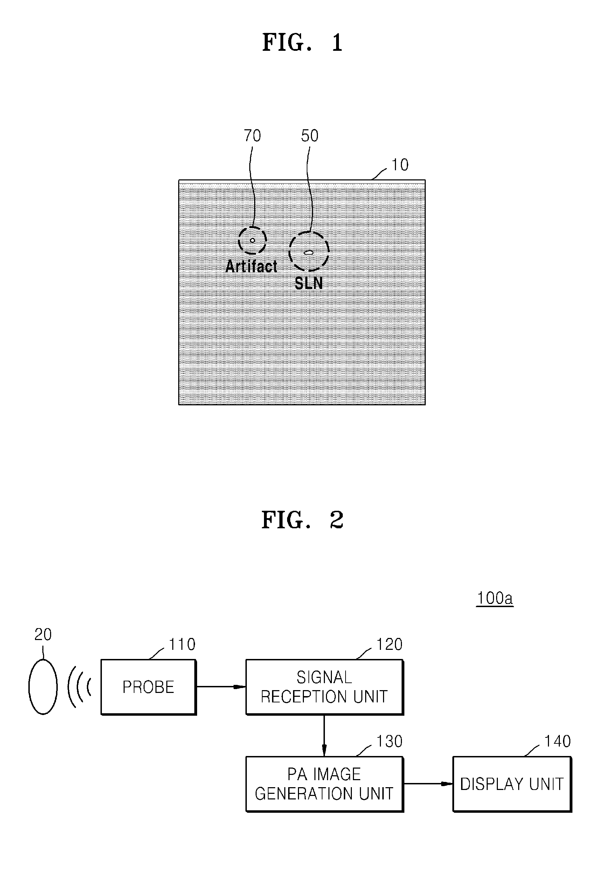

FIG. 1 illustrates a photoacoustic (PA) image 10 including an artifact 70.

In FIG. 1, the PA image 10 includes a region of interest (ROI) including a sentinel lymph node (SLN) 50.

For example, a PA apparatus may irradiate a laser beam onto the ROI and receive a PA signal corresponding to the irradiated laser beam and may acquire a PA image on the basis of the received PA signal.

Referring to FIG. 1, the PA image 10 may further include the artifact 70 therein besides the SLN 50.

For example, when a laser beam is irradiated on the ROI, an unknown absorber may absorb the irradiated laser beam, and accordingly, a PA signal may be generated. In addition, when the irradiated laser beam is dispersed on an object or in the air and hits a lens of an ultrasound probe, a PA signal may be generated from the lens, reflected from the object, and received by the ultrasound probe.

The undesired PA signal may form the artifact 70 in the PA image 10.

FIG. 2 is a block diagram of a PA apparatus 100a according to an embodiment of the present invention. Referring to FIG. 2, the PA apparatus 100a may include a probe 110, a signal reception unit 120, a PA image generation unit 130, and a display unit 140.

The PA apparatus 100a may be implemented as not only a cart type but also a portable type. Examples of the PA apparatus 100a may include a picture archiving and communication system (PACS) viewer, a smart phone, a laptop computer, a personal digital assistant (PDA), a tablet personal computer (PC), and the like, but the PA apparatus 100a is not limited thereto.

The probe 110 may receive a laser beam generated by a laser module and irradiate the laser beam onto an object 20. The signal reception unit 120 generates PA data by processing a PA signal received from the probe 110 and may include an amplifier (not shown), an analog-to-digital converter (ADC, not shown), a reception delay unit (not shown), and a summing unit (not shown). The amplifier amplifies the PA signal for each channel, and the ADC analog-digital converts the amplified PA signal. The reception delay unit applies a delay time for determining reception directionality to the digital-converted PA signal, and the summing unit may generate PA data by summing PA signals processed by the reception delay unit.

The PA image generation unit 130 may generate a PA image through a scan conversion process on the PA data generated by the signal reception unit 120.

For example, the PA image generation unit 130 may generate a first PA image with respect to an ROI including a flow, wherein the flow is formed by a target including, for example, a lymph flow, a blood flow, a flow of a dodily fluid, or the like but is not limited thereto, and a second PA image with respect to an ROI in which the flow is restricted. In addition, the PA image generation unit 130 may generate a difference image between the first PA image and the second PA image.

In addition, the PA image generation unit 130 may generate a three-dimensional (3D) image through a volume rendering process on volume data. Furthermore, the PA image generation unit 130 may represent various pieces of additional information on the PA image as a text or a graphic. The generated PA image may be stored in a memory (not shown).

The display unit 140 may display the images generated by the PA image generation unit 130. For example, the display unit 140 may display the first PA image, the second PA image, the difference image between the first PA image and the second PA image, and the like.

In addition, the display unit 140 may display not only the image but also various pieces of information processed by the PA apparatus 100a on a screen through a graphic user interface (GUI). The PA apparatus 100a may include two or more display units 140 according to an implementation form.

The display unit 140 may include at least one selected from the group consisting of a liquid crystal display (LCD), a thin film transistor-liquid crystal display (TFT-LCD), an organic light-emitting diode (OLED), a flexible display, a 3D display, and an electrophoretic display.

When the display unit 140 and a user input unit (not shown) are formed in a layer structure as a touch screen, the display unit 140 may be used as an input device capable of inputting information therethrough by a touch of a user, as well as an output device.

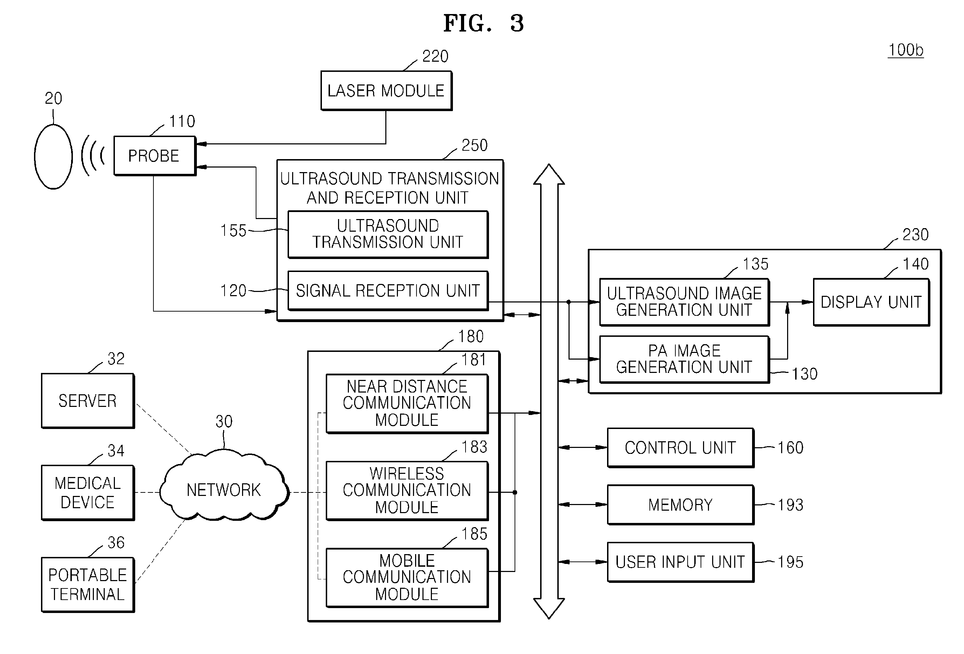

FIG. 3 is a block diagram of a PA apparatus 100b according to another embodiment of the present invention. Referring to FIG. 3, the PA apparatus 100b may include a laser module 220, a probe 110, an ultrasound transmission and reception unit 250, an image processing unit 230, a communication unit 180, a control unit 160, a memory 193, and a user input unit 195, and the image processing unit 230 may include a PA image generation unit 130, an ultrasound image generation unit 135, and a display unit 140.

The probe 110, the signal reception unit 120, the PA image generation unit 130, and the display unit 140 of FIG. 3 are the same as the probe 110, the signal reception unit 120, the PA image generation unit 130, and the display unit 140 of FIG. 2, and thus, a description thereof will not be repeated here.

The probe 110 may emit an ultrasound signal to an object 20 according to a driving signal applied from an ultrasound transmission unit 155 and receive an echo signal reflected from the object 20. The probe 110 includes a plurality of transducers, and the plurality of transducers may vibrate according to a received electrical signal and generate ultrasound waves that carry acoustic energy. In addition, the probe 110 may be connected by wire or wirelessly to a main body of the PA apparatus 100b, and the PA apparatus 100b may include a plurality of probes 110 according to an implementation form.

The ultrasound transmission unit 155 supplies the driving signal to the probe 110 and may include a pulse generation unit (not shown), a transmission delay unit (not shown), and a pulser (not shown). The pulse generation unit may generate pulses for forming transmission ultrasound waves according to a pre-defined pulse repetition frequency (PRF), and the transmission delay unit may apply a delay time for determining transmission directionality to the pulses. The pulses to which the delay time is applied may correspond to a plurality of piezoelectric vibrators (not shown) included in the probe 110, respectively. The pulser may apply the driving signal (or a driving pulse) to the probe 110 at a timing corresponding to each of the pulses to which the delay time is applied.

The signal reception unit 120 may receive not only a PA signal but also an ultrasound echo signal, the amplifier may amplify the signal for each channel, and the ADC may analog-digital convert the amplified signal. The reception delay unit may apply a delay time for determining reception directionality to the digital-converted signal, and the summing unit may generate ultrasound data by summing signals processed by the reception delay unit.

The ultrasound image generation unit 135 may generate an ultrasound image. The ultrasound image may represent not only a gray-scaled ultrasound image obtained by scanning the object 20 according to the A mode, the B mode, or a motion (M) mode but also a motion of the object 20 as a Doppler image. The Doppler image may include a blood stream Doppler image (also called a color Doppler image) representing a flow of blood, a tissue Doppler image representing a motion of tissue, and a spectral Doppler image representing a moving speed of the object 20 as a waveform.

The ultrasound image generation unit 135 may include a B mode processing unit (not shown) and a Doppler processing unit (not shown). The B mode processing unit may extract a B mode component from ultrasound data and process the extracted B mode component. The ultrasound image generation unit 135 may generate an ultrasound image in which the intensity of a signal is represented as brightness, on the basis of the B mode component extracted by the B mode processing unit.

Likewise, the Doppler processing unit may extract a Doppler component from the ultrasound data, and the ultrasound image generation unit 135 may generate a Doppler image in which a motion of the object 20 is represented as a color or a waveform, on the basis of the extracted Doppler component.

The communication unit 180 communicates with an external device or server 32 by being connected by wire or wirelessly to a network 30. The communication unit 180 may exchange data with a hospital server (not shown) or another medical device (not shown) inside the hospital server, which is connected through a PACS. In addition, the communication unit 180 may perform data communication under a digital imaging and communications in medicine (DICOM) standard.

The communication unit 180 may transmit and receive not only data related to diagnosis of the object 20, such as an ultrasound image, a PA image, ultrasound data, Doppler data, and the like of the object 20, but also medical images captured by other medical devices, such as computer tomography (CT), magnetic resonance imaging (MRI), X-ray devices, and the like, through the network 30. Furthermore, the communication unit 180 may receive information regarding a diagnosis history, a therapy schedule, and the like of a patient from the server 32 and allow a user to use the information for diagnosis of the object 20. Also, the communication unit 180 may perform data communication with not only the server 32 and a medical device 34 in a hospital but also a portable terminal 36 of a medical practitioner or a patient.

The communication unit 180 may exchange data with the server 32, the medical device 34, or the portable terminal 36 by being connected by wire or wirelessly to the network 30. The communication unit 180 may include one or more components, e.g., a near distance communication module 181, a wired communication module 183, and a mobile communication module 185, capable of communicating with an external device.

The near distance communication module 181 indicates a module for near distance communication within a pre-defined distance. Near distance communication technology according to an embodiment of the present invention may include wireless local area network (LAN), Wi-Fi, Bluetooth, Zigbee, Wi-Fi Direct (WFD), ultra wideband (UWB), infrared data association (IrDA), Bluetooth low energy (BLE), near field communication (NFC), and the like but is not limited thereto.

The wired communication module 183 indicates a module for communication using an electrical signal or an optical signal, and wired communication technology according to an embodiment of the present invention may include pair cable, coaxial cable, optical fiber cable, Ethernet cable, and the like.

The mobile communication module 185 transmits and receives a wireless signal to and from at least one selected from the group consisting of a base station, an external terminal, and a server in a mobile communication network. The wireless signal may include a voice call signal, a video call signal, or various types of data according to text/multimedia message transmission and reception.

The memory 193 stores various types of information processed by the PA apparatus 100b. For example, the memory 193 may store medical data related to diagnosis of the object 20, such as input/output ultrasound data, ultrasound images, and the like and may also store an algorithm and a program executed inside the PA apparatus 100b.

The memory 193 may be implemented by various types of storage media, such as a flash memory, a hard disk, an electrically erasable programmable read only memory (EEPROM), and the like. In addition, the PA apparatus 100b may operate web storage or a cloud server for performing a storage function of the memory 193 on the web.

The user input unit 195 generates input data according to an input of the user for controlling an operation of the PA apparatus 100b. The user input unit 195 may include hardware components, such as a keypad (not shown), a mouse (not shown), a touch pad (not shown), a track ball (not shown), a jog switch (not shown), and the like, but is not limited thereto. The user input unit 195 may further include various components, such as an electrocardiogram measurement module (not shown), a breathing measurement module (not shown), a voice recognition sensor (not shown), a gesture recognition sensor (not shown), a fingerprint recognition sensor (not shown), an iris recognition sensor (not shown), a depth sensor (not shown), a distance sensor (not shown), and the like.

The control unit 160 controls the general operation of the PA apparatus 100b. That is, the control unit 160 may control operations among the probe 110, the ultrasound transmission and reception unit 250, the image processing unit 230, the communication unit 180, the memory 193, and the user input unit 195.

Some or all of the probe 110, the ultrasound transmission unit 155, the signal reception unit 120, the ultrasound image generation unit 135, the PA image generation unit 130, the control unit 160, the communication unit 180, the memory 193, and the user input unit 195 may operate via a software module but are not limited thereto, and some of the components described above may operate via hardware.

The block diagram of the PA apparatus 100a or 100b illustrated in FIG. 2 or 3 is a block diagram for an embodiment of the present invention. The components in each block diagram may be integrated, added or omitted according to specifications of an actually implemented PA apparatus. That is, two or more components may be integrated as one component, or one component may be divided into two or more components, according to circumstances. In addition, the function performed by each block is to describe an embodiment of the present invention, and a detailed operation or device each block does not limit the rights scope of the present invention.

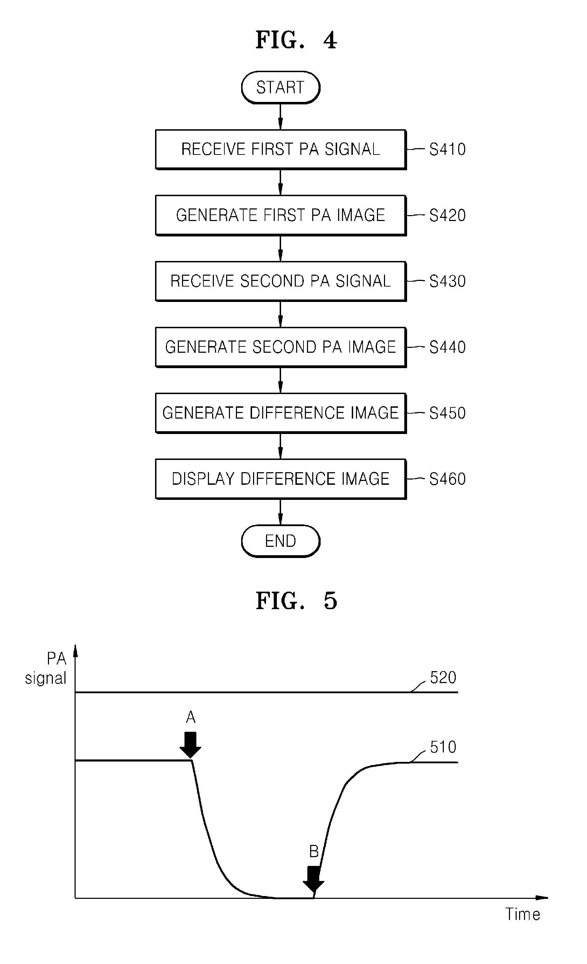

FIG. 4 is a flowchart of a method of operating the PA apparatus 100a or 100b, according to an embodiment of the present invention.

Hereinafter, a method of acquiring a PA image with respect to an SLN will be described as an example for convenience of description. However, the current embodiment is not limited thereto, and the method of operating a PA apparatus in FIG. 4 may be applied to a method of acquiring a PA image with respect to an ROI including a flow instead of the SLN.

Referring to FIG. 4, the PA apparatus 100a or 100b irradiates a laser beam onto an ROI including a flow and receives a first PA signal corresponding to the irradiated laser beam in operation S410.

For example, the PA apparatus 100a or 100b may irradiate a laser beam onto the ROI including a flow, such as an SLN, and receive the first PA signal.

The PA apparatus 100a or 100b generates a first PA image on the basis of the received first PA signal in operation S420.

The PA apparatus 100a or 100b irradiates a laser beam onto the ROI in which the flow is restricted and receives a second PA signal corresponding to the irradiated laser beam in operation S430. For example, a user may restrict the flow of the SLN, irradiate a laser beam onto the ROI in which the flow is restricted, and receive the second PA signal.

The PA apparatus 100a or 100b generates a second PA image on the basis of the received second PA signal in operation S440.

A magnitude of a PA signal with respect to an ROI including a flow may be proportional to a flow volume. That is, when the flow volume is large, the PA signal may increase, and when the flow volume is small, the PA signal may decrease.

Accordingly, with respect to the magnitude of the PA signal corresponding to an SLN including a flow, a case where the flow of the SLN is not restricted (the first PA signal) may differ from a case where the flow of the SLN is restricted (the second PA signal).

The difference between the first PA signal and the second PA signal will now be described with reference to FIG. 5.

FIG. 5 illustrates PA signals with respect to time, which correspond to an SLN and an artifact.

Reference numeral 510 indicates a graph showing a PA signal with respect to time which corresponds to the SLN, and reference numeral 520 indicates a graph showing a PA signal with respect to time which corresponds to the artifact.

Referring to FIG. 5, the symbol A indicates a point of time from when a flow starts to be restricted. For example, A may indicate a point of time when a cuff operates. When lymph (flow) flowing through the SLN is restricted by operating the cuff, a magnitude of a received PA signal decreases.

The symbol B may indicate a point of time when the operation of the cuff stops. When the operation of the cuff stops, the restricted lymph (flow) flows through the SLN again, and accordingly, a magnitude of the PA signal increases.

Accordingly, the first PA signal in a case where the flow is not restricted may differ in the magnitude from the second PA signal in a case where the flow is restricted.

On the contrary, the PA signal corresponding to the artifact without including the flow may be constantly maintained even though the flow in the ROI is restricted.

Referring back to FIG. 4, the PA apparatus 100a or 100b generates a difference image between the first PA image and the second PA image in operation S450.

For example, the PA apparatus 100a or 100b may generate the first PA image on the basis of a PA signal received by irradiating a laser beam onto the ROI before the point of time A when the cuff operates or a PA signal received by irradiating a laser beam onto the ROI after the point of time B when the operation of the cuff stops as shown in FIG. 5.

In addition, the PA apparatus 100a or 100b may generate the second PA image on the basis of a PA signal received by irradiating a laser beam onto the ROI between the point of time A when the cuff operates and the point of time B when the operation of the cuff stops as shown in FIG. 5.

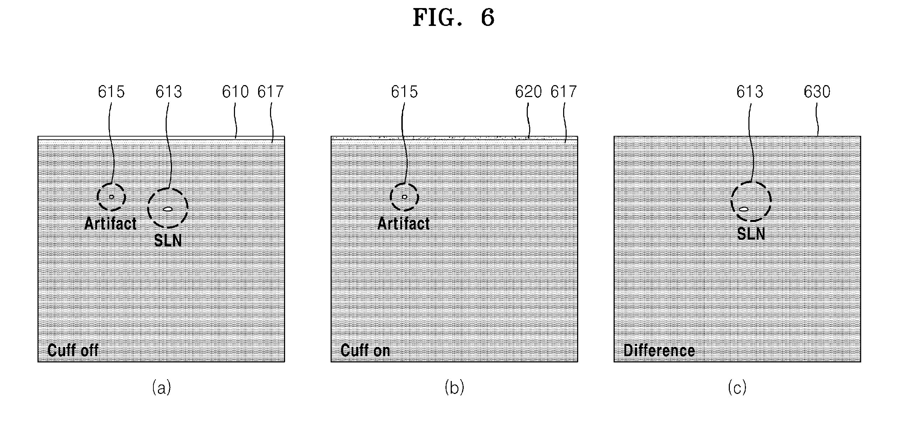

FIGS. 6A to 6C illustrate a first PA image 610, a second PA image 620, and a difference image 630, respectively, according to an embodiment of the present invention.

FIG. 6A shows a PA image (the first PA image 610) when a flow is not limited (for example, cuff off), and FIG. 6B shows a PA image (the second PA image 620) when the flow is limited (for example, cuff on).

As shown in FIGS. 6A and 6B, the first PA image 610 includes a PA image 613 with respect to an SLN and artifact images 615 and 617, but the second PA image 620 includes only the artifact images 615 and 617 without the PA image 613 with respect to the SLN according to a decrease in the magnitude of a PA signal with respect to the SLN.

FIG. 6C shows the difference image 630 between the first PA image 610 and the second PA image 620. The difference image 630 may be an image which includes only the PA image 613 with respect to the SLN and from which the artifact images 615 and 617 have been removed.

For example, the PA image in FIG. 6C may be an image from which the artifact image 617 due to a lens, or the artifact image 615 due to an unknown absorber included in FIGS. 6A and 6B, have been removed.

Referring back to FIG. 4, the PA apparatus 100a or 100b displays the difference image on the display unit 140 in operation S460.

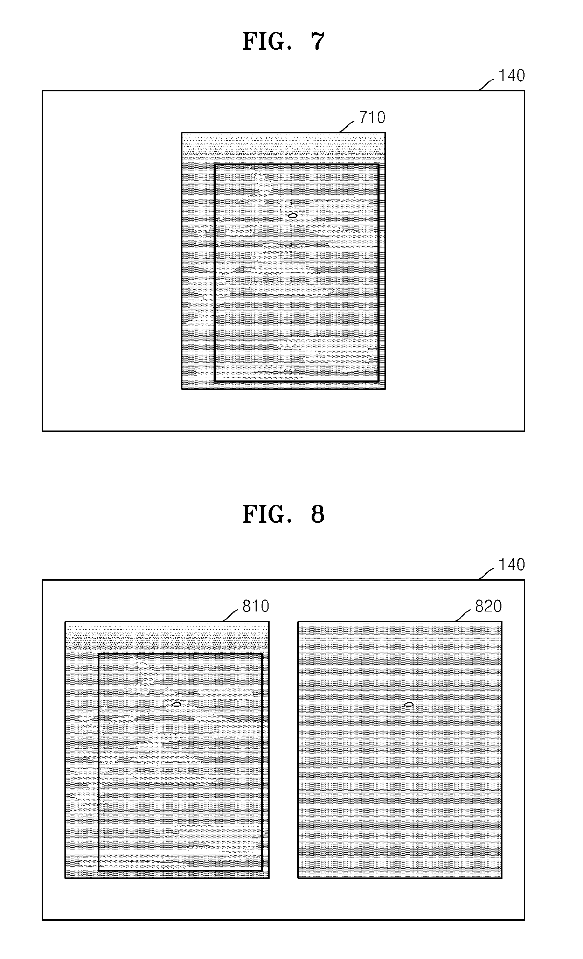

For example, FIGS. 7 to 9 illustrate a PA image displayed on the display unit 140.

Referring to FIG. 7, one screen 710 may be displayed on the display unit 140, and an image in which an ultrasound image and the first PA image overlap each other or an image in which the ultrasound image and the difference image overlap each other may be displayed on the screen.

The ultrasound image may be a B mode image but is not limited thereto. Unlike a PA image, the ultrasound image may image a biological structure, showing, for example, a position, a shape, and the like, and biomechanical properties of a target inside an object, and thus, when the ultrasound image and a PA image overlap and are simultaneously displayed, the user may acquire more information than when any one thereof is displayed.

In addition, although not shown, images in which a first order differential value and a second order differential value of a difference between the first PA signal and the second PA signal are visually represented may be displayed. In addition, a magnitude difference between the first PA signal and the second PA signal, the first order differential value, and the like may be displayed with different colors according to a rate of change.

Referring to FIG. 8, first and second screens 810 and 820 may be displayed on the display unit 140, wherein the image in which the ultrasound image and the first PA image overlap each other is displayed on the first screen 810, and the image in which the ultrasound image and the difference image overlap each other is displayed on the second screen 820.

Alternatively, the ultrasound image may be displayed on the first screen 810, and the difference image may be displayed on the second screen 820.

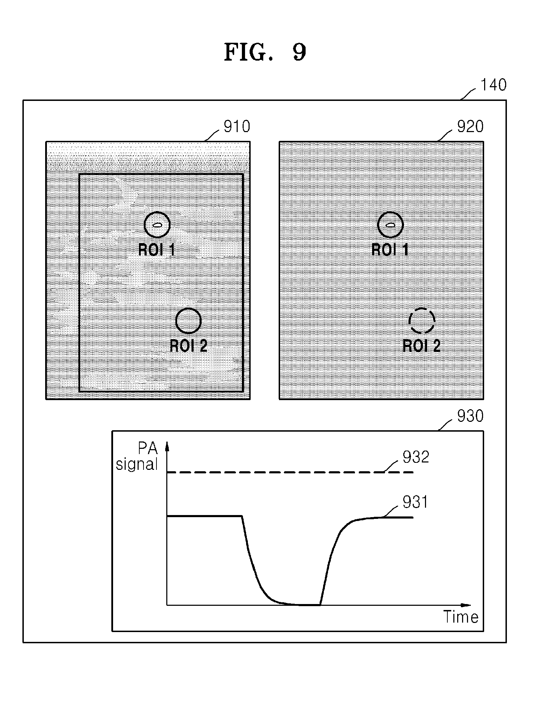

Referring to FIG. 9, first, second, and third screens 910, 920, and 930 may be displayed on the display unit 140, wherein the image in which the ultrasound image and the first PA image overlap each other is displayed on the first screen 910, and the difference image is displayed on the second screen 920.

Alternatively, the ultrasound image may be displayed on the first screen 910, and the difference image may be displayed on the second screen 920.

In addition, a graph showing a magnitude of PA signals with respect to time, with respect to ROIs selected by the user may be displayed on the third screen 930.

For example, when the user selects a first ROI ROI1 and a second ROI ROI2 in an image displayed on the first or second screen 910 or 920, a change in a magnitude of a PA signal with respect to time, with respect to the first ROI ROI1 and a change in a magnitude of a PA signal with respect to time, with respect to the second ROI ROI2 may be displayed on the third screen 930.

For example, in FIG. 9, reference numeral 931 indicates a graph showing a magnitude with respect to time, with respect to a PA signal corresponding to the first ROI ROI1, and reference numeral 932 indicates a graph showing a magnitude with respect to time, with respect to a PA signal corresponding to the second ROI ROI2.

Accordingly, the user may estimate, as an artifact, the image shown in the second ROI ROI2 for which a magnitude of a PA signal is not changed with respect to time as shown in FIG. 9. In addition, the user may estimate an image, which is not shown in the difference image, as an artifact image.

The PA apparatus and the method of operating the same can also be embodied as computer-readable codes on a computer-readable recording medium. The computer-readable recording medium is any data storage device that can store data which can be thereafter read by a computer system. Examples of the computer-readable recording medium include read-only memory (ROM), random-access memory (RAM), CD-ROMs, magnetic tapes, floppy disks, and optical data storage devices. The computer-readable recording medium can also be distributed over network coupled computer systems so that the computer-readable code is stored and executed in a distributed fashion.

In addition, other embodiments of the present invention can also be implemented through computer-readable code/instructions in/on a medium, e.g., a computer-readable medium, to control at least one processing element to implement any of the above described embodiments. The medium can correspond to any medium/media permitting the storage and/or transmission of the computer-readable code.

The computer-readable code can be recorded/transferred on a medium in a variety of ways, with examples of the medium including recording media, such as magnetic storage media (e.g., ROM, floppy disks, hard disks, etc.) and optical recording media (e.g., CD-ROMs, or DVDs), and transmission media such as Internet transmission media. Thus, the medium may be such a defined and measurable structure including or carrying a signal or information, such as a device carrying a bitstream according to one or more embodiments of the present invention. The media may also be a distributed network, so that the computer-readable code may be stored/transferred and executed in a distributed fashion. Furthermore, the processing element could include a processor or a computer processor, and processing elements may be distributed and/or included in a single device.

It should be understood that the exemplary embodiments described herein should be considered in a descriptive sense only and not for purposes of limitation. Descriptions of features or aspects within each embodiment should typically be considered as available for other similar features or aspects in other embodiments.

While one or more embodiments of the present invention have been described with reference to the figures, it will be understood by those of ordinary skill in the art that various changes in form and details may be made therein without departing from the spirit and scope of the present invention as defined by the following claims.

## Claims

1. A method of operating a photoacoustic (PA) apparatus, the method comprising:
irradiating a laser beam onto a region of interest (ROI) which includes a flow and receiving a first PA signal corresponding to the irradiated laser beam;
generating a first PA image based on the first PA signal;
irradiating a laser beam onto the ROI where the flow is restricted and receiving a second PA signal corresponding to the irradiated laser beam;
generating a second PA image based on the second PA signal;
generating a difference image between the first PA image and the second PA image; and
displaying the difference image.

2. The method of claim 1, wherein magnitudes of the first PA signal and the second PA signal are proportional to an amount of the flow.

3. The method of claim 1, wherein the first PA signal includes a signal corresponding to an artifact and a signal corresponding to the flow.

4. The method of claim 1, wherein the second PA signal includes a signal corresponding to an artifact.

5. The method of claim 4, wherein the second PA image is an artifact image.

6. The method of claim 5, wherein the difference image is an image from which the artifact image has been removed.

7. The method of claim 1, further comprising:
transmitting an ultrasound signal to the ROI and receiving an echo signal reflected from the ROI; and
generating an ultrasound image based on the echo signal.

8. The method of claim 7, wherein the displaying of the difference image comprises overlapping and displaying the difference image and the ultrasound image.

9. A photoacoustic (PA) apparatus comprising:
a probe for irradiating a laser beam onto a region of interest (ROI) which includes a flow;
a signal reception unit for receiving a first PA signal corresponding to the laser beam irradiated onto the ROI which includes the flow and receiving a second PA signal corresponding to the laser beam irradiated onto the ROI where the flow is restricted;
an image generation unit for generating a first PA image based on the first PA signal, generating a second PA image based on the second PA signal, and generating a difference image between the first PA image and the second PA image; and
a display unit for displaying the difference image.

10. The PA apparatus of claim 9, wherein magnitudes of the first PA signal and the second PA signal are proportional to an amount of the flow.

11. The PA apparatus of claim 9, wherein the first PA signal includes a signal corresponding to an artifact and a signal corresponding to the flow.

12. The PA apparatus of claim 9, wherein the second PA signal includes a signal corresponding to an artifact.

13. The PA apparatus of claim 9, wherein the difference image is an image from which an artifact has been removed.

14. The PA apparatus of claim 9, wherein the probe transmits an ultrasound signal to the ROI,
the signal reception unit receives an echo signal reflected from the ROI, and
the PA apparatus further comprises an ultrasound image generation unit for generating an ultrasound image based on the echo signal.

15. The PA apparatus of claim 14, wherein the display unit overlaps and displays the difference image and the ultrasound image.
